(19) Europäisches Patentamt
European Patent Office
Office européen des brevets

(11) Veröffentlichungsnummer: **0 021 373**
**B1**

(12) # EUROPÄISCHE PATENTSCHRIFT

(45) Veröffentlichungstag der Patentschrift: **07.07.82**

(21) Anmeldenummer: **80103481.0**

(22) Anmeldetag: **21.06.80**

(51) Int. Cl.³: **C 07 C 51/60**, C 07 C 63/15, C 07 C 63/22, C 07 C 63/24, C 07 C 63/30, C 07 C 63/38 // B01J31/02

(54) **Verfahren zur Herstellung aromatischer Dicarbonsäuredichloride und ihre Verwendung bei der Herstellung von Polykondensaten.**

(30) Priorität: **03.07.79 DE 2926736**

(43) Veröffentlichungstag der Anmeldung: **07.01.81 Patentblatt 81/1**

(45) Bekanntmachung des Hinweises auf die Patenterteilung: **07.07.82 Patentblatt 82/27**

(84) Benannte Vertragsstaaten: **BE DE FR GB IT NL**

(56) Entgegenhaltungen:
**EP-A-0 013 196**
**DE-A-2 240 883**
**GE-A-401 643**
**US-A-3 318 950**
**US-A-4 129 594**

(73) Patentinhaber: **BAYER AG, Zentralbereich Patente, Marken und Lizenzen, D-5090 Leverkusen 1, Bayerwerk (DE)**

(72) Erfinder: **Freitag, Dieter, Dr., Hasenheide 10, D-4150 Krefeld (DE)**
Erfinder: **Bottenbruch, Ludwig, Dr., Wöhlerstrasse 5, D-4150 Krefeld (DE)**
Erfinder: **Wulff, Claus, Dr., Kaiserstrasse 248, D-4150 Krefeld (DE)**

**EP 0 021 373 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung aromatischer Dicarbonsäuredichloride und ihre Verwendung bei der Herstellung von Polykondensaten

Die Erfindung betrifft ein Einstufen-Verfahren zur Herstellung von hochreinen polykondensationsfähigen aromatischen Dicarbonsäuredichloriden.

Die Erzeugung von aliphatischen und aromatischen Säurechloriden durch Reaktion einer Carbonsäure mit Phosgen ist in den US-PS 3184506, 3544626, 3544627, 3547960 sowie in der DE-OS 2400007 beschrieben. Nach diesen Verfahren werden als Reaktionsprodukte dunkelgefärbte Carbonsäurechloride in einer Reinheit von 96 bis 99% erhalten. Aromatische Dicarbonsäuredichloride dieses geringen Reinheitsgrades können nicht direkt nach dem Zweiphasengrenzflächenpolykondensationsverfahren zur Herstellung von hochmolekularen Polykondensaten, wie aromatischen Polyamiden oder aromatischen Polyestern, eingesetzt werden. Ihr Gehalt an nicht oder nur halbseitig umgesetzten Dicarbonsäuren stört die Polykondensation, verursacht Kettenabbruch und ergibt Polymerisate mit endständigen Carboxylgruppen. Die so hergestellten aromatischen Dicarbonsäuredichloride sind durch Verunreinigungen dunkel gefärbt und enthalten durch Umsetzung mit den Katalysatoren entstandene störende Carbamidsäurechloride (vergl. Chem. Ref. 1973, Vol. 73, Nr. 1, Seite 77 oder Angewandte Chemie (1974), Jahrg. 1962, Nr. 21, Seite 864).

Um farblose Dicarbonsäuredichloride zu erhalten, müssen die Rohprodukte durch Umkristallisation oder Destillation gereinigt werden. Dies erfordert zusätzlichen Aufwand und vermindert die Ausbeute; bei aromatischen Dicarbonsäuredichloriden besteht die Gefahr einer spontanen Zersetzung.

Gegenstand der Erfindung ist ein Einstufen-Verfahren zur Herstellung von reinen, aromatischen Dicarbonsäuredichloriden durch Umsetzung einer aromatischen Dicarbonsäure oder eines aromatischen Dicarbonsäuregemisches mit Phosgen in Anwesenheit eines Katalysators, und gegebenenfalls in einem Lösungs- oder Verdünnungsmittel, das dadurch gekennzeichnet ist, dass man als Katalysatoren N-$C_1$–$C_6$-Alkyl-Pyrrolidine, N-$C_1$–$C_6$-Alkyl-Piperidine oder N-$C_1$–$C_6$-Alkyl-Morpholine einsetzt und diese nach der Umsetzung destillativ entfernt. Die so erhaltenen aromatischen Dicarbonsäuredichloride sind praktisch farblos und enthalten 0,1% Verunreinigungen oder weniger, so dass sie ohne Nachreinigung zur Herstellung von farblosen, hochmolekularen Polykondensaten eingesetzt werden können.

Die erfindungsgemäss eingesetzten Katalysatoren können durch Andestillation des Reaktionsgemisches nahezu quantitativ entfernt werden. Katalysatorreste beeinflussen die Umsetzung der aromatischen Dicarbonsäuredichloride zu hochmolekularen Polykondensaten nicht.

Erfindungsgemässe Katalysatoren sind:
N-$C_1$–$C_6$-Alkyl-Pyrrolidine, -Piperidine und -Morpholine. $C_1$–$C_6$-Alkyl bedeutet insbesondere Methyl-, Ethyl-, Propyl-, Isopropyl-, Butyl-, sek.-Butyl-, und tert.-Butyl-, sowie N-Pentyl und N-Hexyl. Besonders geeignet sind N-Ethylpyrrolidin, N-Ethyl-piperidin, N-Ethyl-morpholin, N-Isopropylpiperidin, N-Isopropylmorpholin.

Im allgemeinen werden 0,2 bis 3 Gew.-%, bevorzugt 0,5 bis 1,5 Gew.-% Katalysator, bezogen auf die eingesetzten aromatischen Dicarbonsäuren, eingesetzt. Aromatische Dicarbonsäuren entsprechen den folgenden Formeln:

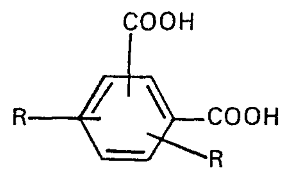
(I)

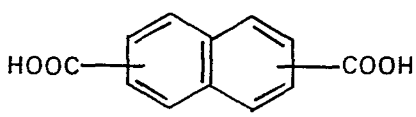
(II)

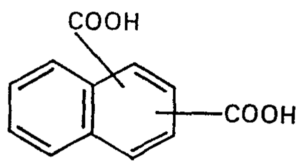
(III)

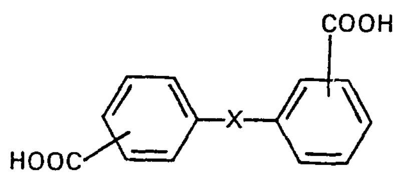
(IV)

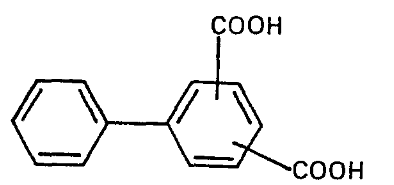
(V)

mit R = H, $C_1$–$C_4$-Alkyl oder Halogen (bevorzugt Chlor und Brom)
X = einfache Bindung, -O-, -S-,

$-CH_2-$, $-\overset{CH_3}{\underset{CH_3}{C}}-$, $C_5$–$C_7$Cycloalkylen.

Auch Gemische können verwendet werden.

Beispielsweise seien genannt:
Phthalsäure, Isophthalsäure, Terephthalsäure, Gemische aus Iso- und Terephthalsäure, Diphen-

säure und 1,4-Naphthalindicarbonsäure.

Als Lösungs- oder Verdünnungsmittel werden vorzugsweise das oder die (bei Gemischen) aromatischen Dicarbonsäuredichloride eingesetzt, die während der Reaktion gebildet werden. Inerte Verdünnungsmittel, wie aliphatische oder aromatische Kohlenwasserstoffe, Halogen-substituierte aromatische Kohlenwasserstoffe, Halogen-substituierte aliphatische Kohlenwasserstoffe oder gesättigte aliphatische Ether, können mitverwendet werden. Die Reaktionstemperatur ist im allgemeinen 110 bis 180°C, vorzugsweise 140 bis 160°C.

Das Mol-Verhältnis aromatische Dicarbonsäure zu Phosgen ist bevorzugt 1:2 bis 1:2,5, d.h., ein geringer Überschuss an Phosgen ist ratsam, um Verluste zu ersetzen, die bei der Austreibung von $CO_2$- und HCl-Gas aus dem Reaktionsgemisch während der Phosgenierung entstehen.

Das erfindungsgemässe Verfahren kann diskontinuierlich oder kontinuierlich ausgeführt werden. In einer kontinuierlichen Ausführungsform lässt man in einem Reaktionsrohr eine Lösung aus aromatischer Dicarbonsäure, Dicarbonsäuredichlorid und Katalysator nach unten gegen aufströmendes Phosgengas strömen und nimmt am Boden des Reaktionsrohres aromatisches Dicarbonsäuredichlorid und Katalysator ab.

In einer diskontinuierlichen Ausführungsform werden unter Normaldruck aromatische Dicarbonsäure, aromatisches Dicarbonsäuredichlorid und Katalysator vorgelegt. Dann wird unter Rühren auf 140 bis 160°C erhitzt, wobei sich die aromatische Dicarbonsäure ganz oder teilweise löst. Bei dieser Temperatur werden pro Mol aromatischer Dicarbonsäure 2 bis 2,5 Mol gasförmiges Phosgen eingeleitet.

Nach Entfernen von überschüssigem Phosgen, HCl- und $CO_2$-Gas sowie eines Teiles oder des gesamten Katalysators durch kurzzeitiges Anlegen von Vakuum erhält man einen Rückstand, der zu $\geqq$ 99,9% aus aromatischem Dicarbonsäuredichlorid besteht, und der ohne Nachreinigung zu hochmolekularen farblosen Polykondensaten umgesetzt werden kann.

Beispiel 1

203 g (1 Mol) Isophthalsäuredichlorid, 166 g Isophthalsäure (1 Mol) und 2,5 ml (2,06 g N-Ethylpiperidin werden im Rundkolben, der mit einem Thermometer, Rührer und einem durch Kühlsole auf -20°C gehaltenen Intensivkühler ausgestattet ist, auf 149°C erhitzt. Bei 149 bis 158°C wird unter Rühren so lange Phosgen eingeleitet (~240 g), bis ein Temperaturabfall im Reaktionsgemisch (auf ~145°C) eintritt. Bei weiterer Phosgeneinleitung siedet dieses unter Rückfluss.

Nach Abkühlen auf 120°C wird Wasserstrahlvakuum angelegt, wodurch überschüssiges Phosgen sowie im Reaktionsgemisch gelöstes HCl- und $CO_2$-Gas und die Hauptmenge an Katalysator entfernt werden.

Man erhält 406 g eines nahezu farblosen Rückstandes mit einer Reinheit von 99,9% an Isophthalsäuredichlorid (titrimetrisch bestimmt).

COOH: $<$ 0,07%.

Beispiel 2

101,5 g (0,5 Mol) Isophthalsäuredichlorid, 101,5 g (0,5 Mol) Terephthalsäuredichlorid, 83 g (0,5 Mol) Isophthalsäure und 83 g (0,5 Mol) Terephthalsäure und 2,5 ml (2,06 g) N-Ethylpiperidin werden, wie in Beispiel 1 beschrieben, mit Phosgen umgesetzt.

Man erhält 405,5 g eines nahezu farblosen Rückstandes eines Gemisches aus Iso- und Terephthalsäuredichlorid mit einer Reinheit von 99,88%.

Beispiel 3

Ausführung wie in Beispiel 2, aber mit 1,25 ml (1,03 g) N-Ethylpiperidin ($\triangleq$ halber Katalysatormenge im Vergleich zu Beispiel 2).

Phosgenierzeit: 7 Stunden

Ausbeute: 405 g eines nahezu farblosen Gemisches aus Iso- und Terphthalsäuredichlorid mit einer Reinheit von 99,94%.

N: 49-54 ppm $\triangleq$ 0,05% N-Ethylpiperidin
COOH: $<$ 0,02%.

Beispiel 4

203 g (1 Mol) Isophthalsäuredichlorid, 203 g (1 Mol) Terephthalsäuredichlorid, 166 g Isophthalsäure (1 Mol), 166 g (1 Mol) Terephthalsäure und 2,5 ml (2,06 g) N-Ethylpiperidin werden mit 470 g Phosgen, wie unter Beispiel 1 angegeben, phosgeniert. Nach vollständiger Phosgeneinleitung werden 166 g (1 Mol) Isophthalsäure und 166 g (1 Mol) Terephthalsäure zugegeben und ohne zusätzlichen Katalysator wird mit weiteren 460 g Phosgen bei 146 bis 154°C innerhalb von ~10 Stunden phosgeniert.

Nach Anlegen von Wasserstrahlvakuum bei 120°C und Entfernen der flüchtigen Anteile erhält man 1216 g eines nahezu farblosen Gemisches aus Iso- und Terephthalsäuredichlorid mit einer Reinheit von 99,9%.

Beispiel 5

Ausführung wie in Beispiel 2, nur mit 2,06 g N-Isopropylmorpholin als Katalysator.

Ausbeute: 405 g eines nahezu farblosen Gemisches aus Iso- und Terephthalsäuredichlorid mit einer Reinheit von 99,9%.

Beispiel 6

Ausführung wie in Beispiel 2, nur mit 2,06 g N-Ethylpyrrolidin als Katalysator.

Ausbeute: 406 g eines nahezu farblosen Gemisches aus Iso- und Terephthalsäuredichlorid mit einer Reinheit von 99,88%.

**Patentansprüche**

1. Einstufen-Verfahren zur Herstellung von reinen, aromatischen Dicarbonsäuredichloriden durch Umsetzung einer aromatischen Dicarbonsäure oder eines aromatischen Dicarbonsäuregemisches mit Phosgen in Anwesenheit eines Katalysators, und gegebenenfalls in einem Lösungs-oder Verdünnungsmittel, dadurch gekennzeichnet, dass man als Katalysatoren N-$C_1$–$C_6$-Alkyl-Pyrrolidine, N-$C_1$–$C_6$-Alkyl-Piperidine oder N-$C_1$–$C_6$-Alkyl-Morpholine einsetzt und diese nach der Umsetzung destillativ entfernt.

2. Verwendung der gemäss Anspruch 1 erhaltenen aromatischen Dicarbonsäuredichloride zur Herstellung von Polykondensaten.

**Claims**

1. A one-shot process for the preparation of pure, aromatic dicarboxylic acid dichlorides by reacting an aromatic dicarboxylic acid or an aromatic dicarboxylic acid mixture with phosgene in the presence of a catalyst, and optionally in a solvent or diluent, characterised in that N-$C_1$–$C_6$-alkyl-pyrrolidines, N-$C_1$–$C_6$-alkyl-piperidines or N-$C_1$–$C_6$-alkyl-morpholines are used as catalysts and these are removed by distillation after the reaction.

2. Use of the aromatic dicarboxylic acid dichlorides obtained according to Claim 1 for the preparation of polycondensates.

**Revendications**

1. Procédé en un stade de fabrication de dichlorures d'acides dicarboxyliques aromatiques purs par réaction d'un acide dicarboxylique aromatique ou d'un mélange d'acides dicarboxyliques aromatiques avec du phosgène en présence d'un catalyseur et éventuellement dans un solvant ou un diluant, caractérisé en ce qu'on utilise comme catalyseurs des N-alcoyl($C_1$–$C_6$)-pyrrolidines, des N-alcoyl($C_1$–$C_6$)-pipéridines ou des N-alcoyl($C_1$–$C_6$)-morpholines et en ce qu'on élimine celles-ci après la réaction par distillation.

2. Utilisation des dichlorures d'acides dicarboxyliques aromatiques obtenus selon la revendication 1 pour la fabrication de polycondensats.